# FASCICULE DE BREVET EUROPEEN

(11) **EP 1 791 942 B1**
(45) Date de publication et mention de la délivrance du brevet: **25.08.2010**
(21) Numéro de dépôt: 05791093.7
(22) Date de dépôt: 18.07.2005
(51) Int. Cl.: C11D 7/50, C23G 5/028

(54) **PROCEDE DE DISSOLUTION D'HUILE A BASSE TEMPERATURE**
VERFAHREN ZUR ÖLLÖSUNG BEI NIEDRIGEN TEMPERATUREN
METHOD FOR DISSOLVING OIL AT LOW TEMPERATURE

(30) Priorité: 29.07.2004 FR 0408363
(43) Date de publication de la demande: 06.06.2007
(73) Titulaire: ARKEMA FRANCE, 92700 Colombes (FR)
(72) Inventeur: LALLIER, Jean-Pierre, F-69720 Saint Bonnet de Mure (FR)
(74) Mandataire: Dang, Doris
(86) Numéro de dépôt international: PCT/FR2005/001827
(87) Numéro de publication internationale: WO 2006/024728

(56) Documents cités:
- WO-A-00/56833
- US-B1- 6 660 709

## Description

La présente invention concerne le domaine des solvants. Elle concerne plus particulièrement un procédé de dissolution d'huile à basse température. La présente invention a également pour objet une nouvelle composition à base de 1,1,1,3,3-pentafluorobutane ( HFC-365mfc) et de 1,1,1,2,3,4,4,5,5,5-decafluoropentane ( HFC- 43-10mee).

Le document WO 00/56833 divulgue des compositions binaires quasi azéotropique consistant essentiellement en 1,1,1,3,3-pentafluorobutane ( HFC-365mfc) et 1,1,1,2,3,4,4,5,5,5-decafluoropentane ( HFC- 43-10mee) ou nonafluoromethoxybutane. Ce document divulgue également des compositions ternaires et quaternaires quasi-azéotropiques consistant essentiellement en 1,1,1,3,3-pentafluorobutane ( HFC-365mfc),1,1,1,2,3,4,4,5,5,5-decafluoropentane (HFC- 43-10mee) ou nonafluoromethoxybutane et trans-1,2-dichloroéthylène (tDCE), bromure de n-propyle, acétone, méthanol, éthanol ou isopropanol.

Les exemples 4 et 5 du document WO 00/56833 decrivent des tests de solubilité d'huile, à température ambiante, de certaines compositions. Ainsi, une composition contenant 31% en poids de 43-10mee, 31% en poids de 365mfc et 38 % en poids de tDCE a été évaluée avec une huile minérale ( Tableau 4) et une composition contenant 33 % en poids de 43-10mee, 28 % en poids de 365mfc et 39 % en poids de tDCE a été évaluée avec une huile silicone DC-200 ( Tableau 5).

Toutefois, les compositions ternaires de ce document ne sont pas efficaces dans des applications nécessitant de travailler à basse température comme par exemple dans le domaine médical ou autre.

La demanderesse a donc mis au point un procédé de dissolution d'huile à basse température.

La présente invention a pour premier objet un procédé de dissolution d'huile à basse température **caractérisé en ce que** l'on utilise une composition comprenant du 1,1,1,3,3-pentafluorobutane ( HFC-365mfc), du 1,1,1,2,3,4,4,5,5,5-decafluoropentane ( HFC- 43-10mee), du trans-1,2-dichloroéthylène ( tDCE ) et de 1 à 10 % en poids d'un alcool contenant de 2 à 4 atomes de carbone.

Le procédé, selon la présente invention, est mis en oeuvre à une température inférieure à 10° C . Une température comprise entre 0 et 8° C est préférée. Une température comprise entre 3 et 6 ° C est avantageusement choisie.

On utilise de préférence une composition comprenant de 40 à 69 % en poids d'un mélange binaire, contenant le 1,1,1,3,3-pentafluorobutane et le1,1,1,2,3,4,4,5,5,5-decafluorobutane, de 30 à 50 % en poids de trans-1,2-dichloroéthylène et de 1 à 10 % en poids d'un alcool contenant de 2 à 4 atomes de carbone.

Avantageusement, on utilise une composition comprenant de 45 à 64 % en poids du mélange binaire HFC- 365mfc et HFC- 43-10mee, de 35 à 45 % en poids de trans-1,2-dichloroéthylène et de 1 à 10 % en poids d'un alcool contenant de 2 à 4 atomes de carbone.

La quantité d'alcool présente dans la composition est avantageusement comprise entre 1 et 5% en poids.

L'éthanol, le propanol, l'isopropanol, le butanol, le second butanol ( sec-butanol) ou le tertiobutanol peuvent convenir. De préférence, on utilise l'isopropanol ou le sec-butanol. Le sec-butanol est avantageusement préféré.

Le 1,1,1,2,3,4,4,5,5,5-decafluoropentane représente, de préférence, au moins 17 % en poids du mélange binaire

Le procédé selon l'invention convient aux huiles minérales ou silicones. Ce procédé peut être appliqué au dépôt uniforme des huiles sur un substrat, par exemple des ustensiles de cuisine. Il convient tout particulièrement au dépôt d'huile de silicone de grade médical, notamment le dépôt sur les instruments médicaux tels que des aiguilles de seringues ou cathéters. En effet, l'huile de silicone est utilisée comme lubrifiant pour les seringues médicales hypodermiques afin de réduire la douleur associée à l'insertion de l'aiguille dans la peau du patient.

Le procédé selon la présente invention convient au nettoyage, dégraissage et séchage de surfaces solides très diverses ( pièces métalliques, verres, plastiques, composites). Il peut également être mis en oeuvre dans la fabrication des circuits imprimés pour éliminer les résidus des substances utilisées pour améliorer la qualité des soudures. Cette opération d'élimination est désignée dans le métier par le terme de « défluxage ».

La présente invention a pour second objet une composition comprenant du 1,1,1,3,3-pentafluorobutane ( HFC-365mfc), du 1,1,1,2,3,4,4,5,5,5-decafluoropentane ( HFC- 43-10mee), du trans-1,2-dichloroéthylène ( tDCE ) et de 1 à 10 % en poids d'un alcool contenant 4 atomes de carbone.

La composition particulièrement préférée, selon le deuxième objet de la présente invention, comprend de 40 à 69 % en poids d'un mélange binaire contenant le 1,1,1,3,3-pentafluorobutane et le1,1,1,2,3,4,4,5,5,5-decafluoropentane, de 30 à 50 % en poids de trans-1,2-dichloroéthylène et de 1 à 10 % en poids d'un alcool contenant 4 atomes de carbone.

Est avantageusement préférée, une composition comprenant de 45 à 64 % en poids du mélange binaire HFC- 365mfc et HFC- 43-10mee, de 35 à 45 % en poids de trans-1,2-dichloroéthylène et de 1 à 10 % en poids d'un alcool contenant 4 atomes de carbone.

La quantité d'alcool présente dans la composition est avantageusement comprise entre 1 et 5% en poids.

Le sec-butanol est avantageusement choisi comme alcool.

Le 1,1,1,2,3,4,4,5,5,5 -decafluoropentane représente, de préférence, au moins 17 % en poids du mélange binaire

La composition, selon le deuxième objet, peut être utilisée dans l'industrie nettoyage, dégraissage et séchage de surfaces solides très diverses ( pièces métalliques, verres, plastiques, composites). Il peut également être mis en oeuvre dans la fabrication des circuits imprimés pour éliminer les résidus des substances utilisées pour améliorer la qualité des soudures. Cette opération d'élimination est désignée dans le métier par le terme de « défluxage ».

En outre, elle peut être utilisée comme agent d'expansion des mousses polyuréthanne, comme agent propulseur d'aérosols, comme fluide caloporteur, comme agent de nettoyage à sec des textiles ou de nettoyage d'installations frigorifiques.

### PARTIE EXPERIMENTALE

### EXEMPLE 1

### Description des essais de solubilisation d'une huile de silicone.

On utilise une huile de silicone Crompton L9000-1000 de la société Crompton Corporation (Greenwich, USA). Il s'agit d'un hydroxypolydimethylsiloxane, liquide transparent ayant une densité de 0,97 à température ambiante (22°C) , un point d'ébullition supérieur à 200°C et un point éclair de 132°C (méthode Pensky-Martens, en coupe fermée).

On prépare des mélanges à température ambiante, c'est-à-dire à 22°C.

Ainsi, dans un flacon de 50 ml, on introduit 18 ml de la composition à base de 365mfc et 43-10mee ( à tester ) et 1,94 g d'huile de silicone, c'est-à-dire qu'on réalise une solution à 10% volumique. On agite ensuite le mélange manuellement pendant 5 minutes.

Une partie du mélange ainsi préparé est maintenue au repos à température ambiante (22°C) et pendant 24 heures. Une autre partie est maintenue au repos à basse température (6°C) pendant 7 jours.

Après la durée de stockage at différentes température, on observe précisément l'aspect du mélange. On considère qu'il y a solubilité à température ambiante ou à 6°C lorsque le mélange est transparent, clair, homogène, monophasique et stable.

Les compositions suivantes ont été préparées:
Composition A : 80 % en poids de HFC- 365mfc et 20 % en poids de HFC - 4310mee
Composition B : 48 % en poids de HFC-365mfc et 12 % en poids de HFC- 4310mee et 40 % en poids de trans-1,2 dichoroéthylène
Composition C : 44 % en poids de HFC-365mfc et 11 % en poids de HFC- 4310mee, 40 % en poids de trans-1,2 dichoroéthylène et 5 % en poids de sec-butanol .

### Résultats

| Composition | Solubilité à 22°C | Solubilité à 6°C |
|---|---|---|
| Composition A | NON | NON |
| Composition B | OUI | NON |
| Composition C | OUI | OUI |

### EXEMPLE 2

### Description du test d'inflammabilité

Pour l'évaluation de l'inflammabilité des compositions, nous avons déterminé leur point éclair selon la méthode normalisée ASTM D3828, coupe fermée SETAFLASH. Le point éclair est la température minimale à laquelle un liquide dégage des vapeurs en quantité suffisante pour former à sa surface un mélange inflammable dans l'air sous l'action d'une source d'ignition, mais sans persistance de flammes au retrait de l'énergie d'activation.

Pour chacune des compositions, nous avons répété 5 fois la mesure afin d'obtenir des résultats fiables.

### Résultats

| Composition | POINT ECLAIR |
|---|---|
| Composition A | NON |
| Composition B | NON |
| Composition C | NON |

### EXEMPLE 3

### Description de la mesure de vitesse d'évaporation

On utilise une grille en acier inoxydable, de forme carrée de 4 cm de côté et possédant 30 fils par cm. Ce type de grille au maillage resserré permet d'obtenir une bonne rétention du solvant après trempage.

On trempe la grille dans un bécher de 100 ml contenant 80 ml de la composition à tester maintenue à température ambiante, sous ventilation contrôlée et stable.

On déclenche un chronomètre qui mesurera la vitesse d'évaporation au moment où on retire la grille totalement immergée de la composition à tester.. La fin de l'évaporation est déterminée en observant le front de composition qui migre au cours du temps (à l'instant de fin d'évaporation, le front disparaît).Une quinzaine de mesures ont été réalisées et la valeur moyenne de ces mesures est reportée dans le tableau ci-après..

### Résultat

| Composition | Temps d'évaporation |
|---|---|
| Composition C | 14 secondes |

## Revendications

1. Procédé de dissolution d'huile à basse température **caractérisé en ce qu'**il est mis en oeuvre à une température inférieure à 10°C avec une composition comprenant du 1,1,1,3,3-pentafluorobutane ( HFC-365mfc ), du 1,1,1,2,3,4,4,5,5,5-decafluoropentane ( HFC- 43-10mee ), du trans-1,2-dichloroéthylène ( tDCE ) et de 1 à 10 % en poids d'un alcool contenant de 2 à 4 atomes de carbone.

2. Procédé selon la revendication 1 **caractérisé en ce que** la température est entre 0 et 8° C et avantageusement entre 3 et 6° C.

3. Procédé selon l'une quelconque des revendications précédentes **caractérisé en ce que** l'on utilise une composition comprenant de 40 à 69 % en poids d'un mélange binaire, contenant le 1,1,1,3,3-pentafluorobutane et le1,1,1,2,3,4,4,5,5,5-decafluorobutane, de 30 à 50 % en poids de trans-1,2-dichloroéthylène et de 1 à 10 % en poids d'un alcool contenant de 2 à 4 atomes de carbone.

4. Procédé selon la revendication 3 **caractérisé en ce que** l'on utilise une composition comprenant de 45 à 64 % en poids du mélange binaire HFC- 365mfc et HFC- 43-10mee, de 35 à 45 % en poids de trans-1,2-dichloroéthylène et de 1 à 10 % en poids d'un alcool contenant de 2 à 4 atomes de carbone.

5. Composition comprenant du 1,1,1,3,3-pentafluorobutane ( HFC-365mfc), du 1,1,1,2,3,4,4,5,5,5-decafluoropentane ( HFC- 43-10mee), du trans-1,2-dichloroéthylène ( tDCE ) et de 1 à 10 % en poids d'un alcool contenant 4 atomes de carbone.

6. Composition selon la revendication 5 **caractérisée en ce qu'**elle comprend de 40 à 69 % en poids d'un mélange binaire, contenant le 1,1,1,3,3-pentafluorobutane et le1,1,1,2,3,4,4,5,5,5-decafluorobutane, de 30 à 50 % en poids de trans-1,2-dichloroéthylène et de 1 à 10 % en poids d'un alcool contenant 4 atomes de carbone.

7. Composition selon la revendication 5 ou 6 **caractérisée en ce qu'**elle comprend de 45 à 64 % en poids du mélange binaire HFC- 365mfc et HFC-43-10mee, de 35 à 45 % en poids de trans-1,2-dichloroéthylène et de 1 à 10 % en poids d'un alcool contenant 4 atomes de carbone.

8. Composition selon la revendication 1 à 7 **caractérisée en** l'alcool est le sec-butanol.

9. Composition selon la revendication 5 à 8 **caractérisée en ce qu'**elle est utilisée dans l'industrie de nettoyage, dégraissage, séchage ou comme agent d'expansion des mousses polyuréthanne, comme agent propulseur d'aérosols, comme fluide caloporteur.

## Claims

1. Process for the dissolution of oil at low temperature, **characterized in that** it is carried out at a temperature of less than 10°C with a composition comprising 1,1,1,3,3-pentafluorobutane (HFC-365mfc), 1,1,1,2,3,4,4,5,5,5-decafluoropentane (HFC-43-10mee), trans-1,2-dichloroethylene (t-DCE ) and from 1 to 10% by weight of an alcohol comprising from 2 to 4 carbon atoms.

2. Process according to Claim 1, **characterized in that** the temperature is between 0 and 8°C and advantageously between 3 and 6°C.

3. Process according to either one of the preceding claims, **characterized in that** use is made of a composition comprising from 40 to 69% by weight of a binary mixture comprising 1,1,1,3,3-pentafluorobutane and 1,1,1,2,3,4,4,5,5,5-decafluorobutane, from 30 to 50% by weight of trans-1,2-dichloroethylene and from 1 to 10% by weight of an alcohol comprising from 2 to 4 carbon atoms.

4. Process according to Claim 3, **characterized in that** use is made of a composition comprising from 45 to 64% by weight of the HFC-365mfc and HFC-43-10mee binary mixture, from 35 to 45% by weight of trans-1,2-dichloroethylene and from 1 to 10% by weight of an alcohol comprising from 2 to 4 carbon atoms.

5. Composition comprising 1,1,1,3,3-pentafluorobutane (HFC-365mfc), 1,1,1,2,3,4,4,5,5,5-decafluoropentane (HFC-43-10mee), trans-1,2-dichloroethylene (t-DCE ) and from 1 to 10% by weight of an alcohol comprising 4 carbon atoms.

6. Composition according to Claim 5, **characterized in that** it comprises from 40 to 69% by weight of a binary mixture comprising 1,1,1,3,3-pentafluorobutane and 1,1,1,2,3,4,4,5,5,5-decafluoropentane, from 30 to 50% by weight of trans-1,2-dichloroethylene and from 1 to 10% by weight of an alcohol comprising 4 carbon atoms.

7. Composition according to Claim 5 or 6, **characterized in that** it comprises from 45 to 64% by weight of the HFC-365mfc and HFC-43-10mee binary mixture, from 35 to 45% by weight of trans-1,2-dichloroethylene and from 1 to 10% by weight of an alcohol comprising 4 carbon atoms.

8. Composition according to Claims 5 to 7, **characterized in that** the alcohol is sec-butanol.

9. Composition according to Claims 5 to 8, **characterized in that** it is used in the cleaning, degreasing or drying industry or as blowing agent for polyurethane foams, as aerosol propellant or as heat-exchange fluid.

## Patentansprüche

1. Verfahren zum Lösen von Öl bei niedriger Temperatur, **dadurch gekennzeichnet, daß** man es bei einer Temperatur von weniger als 10°C mit einer Zusammensetzung, die 1,1,1,3,3-Pentafluorbutan (H-FKW 365mfc), 1,1,1,2,3,4,4,5,5,5-Decafluorpentan (H-FKW 43-10mee), trans-1,2-Dichlorethylen (t-DCE) und 1 bis 10 Gew.-% eines Alkohols mit 2 bis 4 Kohlenstoffatomen umfaßt, durchführt.

2. Verfahren nach Anspruch 1, **dadurch gekennzeichnet, daß** die Temperatur zwischen 0 und 8°C und vorteilhafterweise zwischen 3 und 6°C liegt.

3. Verfahren nach einem der vorhergehenden Ansprüche, **dadurch gekennzeichnet, daß** man eine Zusammensetzung, die 40 bis 69 Gew.-% einer binären Mischung, die 1,1,1,3,3-Pentafluorbutan und 1,1,1,2,3,4,4,5,5,5-Decafluorbutan enthält, 30 bis 50 Gew.-% trans-1,2-Dichlorethylen und 1 bis 10 Gew.-% eines Alkohols mit 2 bis 4 Kohlenstoffatomen umfaßt, verwendet.

4. Verfahren nach Anspruch 3, **dadurch gekennzeichnet, daß** man eine Zusammensetzung, die 45 bis 64 Gew.-% einer binären Mischung von 1H-FKW 365mfc und H-FKW 43-10mee, 35 bis 45 Gew.-% trans-1,2-Dichlorethylen und 1 bis 10 Gew.-% eines Alkohols mit 2 bis 4 Kohlenstoffatomen umfaßt, verwendet.

5. Zusammensetzung, umfassend 1,1,1,3,3-Pentafluorbutan (H-FKW 365mfc), 1,1,1,2,3,4,4,5,5,5-Decafluorpentan (H-FKW 43-10mee), trans-1,2-Dichlorethylen (t-DCE) und 1 bis 10 Gew.-% eines Alkohols mit 4 Kohlenstoffatomen.

6. Zusammensetzung nach Anspruch 5, **dadurch gekennzeichnet, daß** sie 40 bis 69 Gew.-% einer binären Mischung, die 1,1,1,3,3-Pentafluorbutan und 1,1,1,2,3,4,4,5,5,5-Decafluorbutan enthält, 30 bis 50 Gew.-% trans-1,2-Dichlorethylen und 1 bis 10 Gew.-% eines Alkohols mit 4 Kohlenstoffatomen umfaßt.

7. Zusammensetzung nach Anspruch 5 oder 6, **dadurch gekennzeichnet, daß** sie 45 bis 64 Gew.-% einer binären Mischung von H-FKW 365mfc und H-FKW 43-10mee, 35 bis 45 Gew.-% trans-1,2-Dichlorethylen und 1 bis 10 Gew.-% eines Alkohols mit 4 Kohlenstoffatomen umfaßt.

8. Zusammensetzung nach Anspruch 5 bis 7, **dadurch gekennzeichnet, daß** es sich bei dem Alkohol um sec.-Butanol handelt.

9. Zusammensetzung nach Anspruch 5 bis 8, **dadurch gekennzeichnet, daß** sie in der Reinigungs-, Entfettungs- oder Trocknungstechnik oder als Treibmittel für Polyurethanschaumstoffe, als Treibmittel für Aerosole oder als Wärmeübertragungsflüssigkeit verwendet wird.
